# EUROPEAN PATENT APPLICATION

(11) **EP 2 645 099 A1**
(43) Date of publication of application: **02.10.2013**
(21) Application number: 12162548.7
(22) Date of filing: 30.03.2012
(51) Int. Cl.: G01N 33/15, A61K 9/107

(54) **Biorelevant compositions**

(71) Applicant: PHARES PHARMACEUTICAL RESEARCH N.V., Curaçao (AN)
(72) Inventor: The designation of the inventor has not yet been filed
(74) Representative: Hasler, Erich

(57) **Abstract**

Disclosed is a standardised aqueous biorelevant media for simulating fasted state stomach and fasted state upper small intestinal fluids of mammalian species, composed of surfactants occurring in the gastro intestinal tract of mammals comprising (a) at least 40 mole % to 95 mole % of one bile salt, and (b) the rest mole % being a combination of at least two surfactants, namely a diacyl phospholipid and a fatty acid including monovalent salts of the fatty acid, or a monoacyl phospholipid and a fatty acid including monovalent salts of the fatty acid, or a diacyl phospholipid and a monoacyl phospholipid and a fatty acid including monovalent salts of the fatty acid, or a diacyl phospholipid and a monoacyl phospholipid, which further is defined by a surface tension between 25 mN/m and 50 mN/m.

Disclosed is further a homogeneous biorelevant composition for preparing fasted state biorelevant media characterised by a surface tension between 25 mN/m and 50 mN/m comprising the following surfactants: (a) at least 40 mole % to 95 mole % of one bile salt, and (b) the rest mole % being a combination of at least two surfactants, namely a diacyl phospholipid and a fatty acid including monovalent salts of the fatty acid, or a monoacyl phospholipid and a fatty acid including monovalent salts of the fatty acid, or a diacyl phospholipid and a monoacyl phospholipid and a fatty acid including monovalent salts of the fatty acid, or a diacyl phospholipid and a monoacyl phospholipid.

## Description

This invention relates to biorelevant media characterised by physicochemical parameters that closely simulate physiological fluids in the stomach and intestine. The invention discloses homogeneous biorelevant compositions for reconstitution as biorelevant media and a method for reconstituting biorelevant media from the aforesaid compositions. The resulting biorelevant media are suitable for use in solubility, dissolution and further assessments of poorly water soluble compounds and their dosage forms, with a view to oral administration.

### Background to the Invention

### Upper GI Physiology and importance of site of dissolution

Before absorption of a drug from the digestive tract can occur, it must be in solution. For immediate release dosage forms, the first opportunity for release and dissolution is in the stomach. The human stomach functions as a processing organ for food and drugs entering the digestive tract. In the fasted state, humans typically have a low pH in the stomach. Basic drugs, which are ionised at low pH, can be readily dissolved under these conditions and so become available for absorption as soon as they enter the small intestine. In fact, if a basic drug is poorly water soluble, a low pH in the stomach provides the best opportunity in the gastrointestinal tract for dissolution, as the pH rises abruptly upon passage into the small intestine and continues to rise along this region. However, in humans with elevated gastric pH due to intake of gastric acid inhibitors, certain diseases and in a certain percentage of the elderly, as well as in dogs, which often have a close to neutral gastric pH, dissolution of basic drugs may be compromised. This may in turn limit the bioavailability of the drug when administered orally.

For drugs that are non-ionisable, dissolution in the stomach and in the small intestine both contribute to the drug being available for absorption from the small intestine. For acidic drugs, solubility and dissolution will be most reliable at the higher pH of the small intestine. Since the average passage time through the empty stomach is typically under 30 minutes, the extent of dissolution of a poorly soluble acidic or neutral drug is expected to be very low in the stomach. However, acidic drugs with some intrinsic solubility may dissolve appreciably before leaving the stomach.

In the small intestine, the dissolution of poorly soluble, weakly acidic drugs will be supported by the higher pH (approx. 5-8) and further enhanced by the natural surfactants, principally bile salts and phospholipids, which can solubilize the poorly soluble drug in colloidal aggregates, including mixed micelles. This mechanism of increasing the solubility and dissolution is also available to poorly soluble neutrals and weakly basic drugs, in the latter case partly offsetting the less favourable pH conditions. Since the transit along the intestine is about 3 to 5 hours in humans, the time available for release and dissolution of the drug from the dosage form is more favourable in this region than in the fasted stomach. This relation generally applies to other mammalian species with simple stomachs, although the absolute passage times in the stomach and small intestine are naturally different in each species. It is in the small intestine that the quantitative absorption of drugs generally takes place after oral administration of an immediate release dosage form.

### What drugs are regarded as poorly soluble?

The Biopharmaceutical Classification System is a way to classify drugs into 4 categories according to the aqueous solubility and permeability:

| | |
|---|---|
| Class I: | High Solubility - High Permeability |
| Class II: | Low Solubility - High Permeability |
| Class III: | High Solubility - Low Permeability |
| Class IV: | Low Solubility - Low Permeability |

The BCS was developed by Gordon L. Amidon in 1995 (GL Amidon et al., Pharm Res Vol 12(3): 413-420, 1995).

Poorly soluble compounds are classified as class 2 and class 4 compounds in the BCS. For these drugs, solubility and dissolution in the stomach and small intestine are often critical to the bioavailability after oral administration. So it is desirable to test and benchmark solubility and dissolution of BCS Class 2 and 4 drugs in media simulating these regions to assess to what extent their oral absorption will be limited by poor solubility/dissolution. In the pharmaceutical industry it is common practice to test for solubility in biorelevant media such as Fasted State Simulated Intestinal Fluid (FaSSIF) (Dressman et. al., Dissolution testing as a prognostic tool for oral drug absorption: immediate release dosage forms. Pharm. Res. 15:11-22 (1998) as part of routine preclinical assessment of new drug candidates.

### What are biorelevant media?

Biorelevant media are those which aim to reproduce the conditions in the gastrointestinal (GI) tract *in vitro,* so that the behaviour of drugs and dosage forms in the GI tract can be studied in the laboratory. Typically they are used for *in vitro* solubility and dissolution studies but can also be applied to studies of decomposition under GI conditions or for the determination of the permeability characteristics of the drug. Biorelevant media typically comprise solutions of surfactants which are naturally occurring in the GI tract and are adjusted to pH values representative of the local region to be simulated. Typically, biorelevant media are designed to reflect the gastric and intestinal fluids in the fasted or the fed state.

For changes to established drug products or when a generic version of a drug product should be brought on the market, evaluation of potential formulations in biorelevant media is an important tool to identify a bioequivalent formulation and to de-risk the *in vivo* bioequivalence studies. Release studies in biorelevant media can provide better correlation with *in vivo* plasma profiles from PK studies than results in quality control dissolution media for example, compendial media not comprising natural surfactants, principally bile salts and phospholipids. They are also preferred for establishing *in vitro - in vivo correlations* (IVIVC), which can in turn provide accurate forecasts of *in vivo* plasma profiles from *in vitro* data, thereby reducing the number of *in vivo* studies needed to establish clinical performance after changes in formulation are made.

It is recommended that new compounds and generic versions of known drug products are tested not only in biorelevant media representing the intestine but also in biorelevant media representing the stomach. This is especially advantageous for weak bases, which are typically more soluble in acidic conditions. A more comprehensive solubility and dissolution profile of the drug in the stomach as well as in the upper small intestine provides a more complete comparison of *in vitro* with *in vivo* results and paves the way for *in vitro - in silico - in vivo* (IVISIV) modelling and simulation. Further, for formulations which aim to produce a supersaturated concentration of drug in the upper GI tract (e.g. melt extrusion products, salt forms, spray-dried products etc.), the combination of release under biorelevant gastric conditions followed by transfer into biorelevant small intestinal conditions can be used to test for eventual precipitation of the drug after it has been released from the dosage form.

Since it is preferred to develop drug products which can be administered either with or without food, and since for poorly soluble drugs inadequate bioavailability is most frequently associated with administration of the drug product in the fasted state, it is particularly important to evaluate new drug candidates and formulations under conditions that are biorelevant to the fasted state in the stomach and small intestine.

This invention relates to biorelevant media which are standardised in terms of physicochemical properties for example, pH, buffer capacity, osmolality, and in particular for the first time, surface tension within the range defined by selections of bile salt and surfactants simulating fasted state conditions in the stomach and the small intestine. Assessing the solubility and dissolution of new drug candidates in standardised biorelevant in comparison to non-standardised media can help better identify potential problems with inadequate solubility and/or dissolution with respect to making the drug candidate available for absorption. Similarly, evaluation of potential formulations of new drug candidates in standardised media is a more efficient and valuable way to identify the optimal formulation for oral administration.

### Unmet needs

Numerous studies have been reported using biorelevant media for *in vitro* assessments of solubility and dissolution of poorly soluble drugs and prediction of *in vivo* release (see e.g. Shono et al. European Journal of Pharmaceutics and Biopharmaceutics 73 (2009) 107-114; Kleberg review (2010)) However, the currently available biorelevant media have not yet been optimized with respect to a key performance parameter, the surface tension. Another limitation is that *to date* only biorelevant media to simulate the human gastrointestinal tract have been specified. As formulations must also be developed for animal studies in the preclinical phase of drug development, it is especially desirable to have access to biorelevant media which can predict *in vivo* release and dissolution of the drug candidate from the formulation in animal species such as dog, monkey and mini-pig. Furthermore, the biorelevant media representing human gastric conditions must be prepared from scratch in the laboratory - no "instantly" dissolving pre-prepared powders are available commercially. Moreover, there is an unmet need for standardised media which can be implemented with assurance of reproducibility in laboratories around the world. It is advantageous that the media be easily and reproducibly prepared in an efficient manner as this will lead to more reliable results and thereby better forecasting of *in vivo* drug performance.

### Definitions

Biorelevant gastric media for simulating physiological fluids under fasted state conditions in the stomach are referred to forthwith as Fasted State Simulated Gastric Fluids (FaSSGF).

Biorelevant intestinal media for simulating physiological fluids under fasted state conditions in the small intestine are referred to forthwith as Fasted State Simulated Intestinal Fluids (FaSSIF).

"FaSSGF *human"* and "FaSSIF *human",* "FaSSGF *canine"* and "FaSSIF *canine"* are clearly distinguished from prior art FaSSGF and FaSSIF (without suffix), and standardised in terms of physicochemical properties, in particular defined by surface tension (mN/m), molar concentration (mmol/L) of each surfactant, mol ratios between the surfactants (mol %), total amount of all surfactants and optional cosurfactants, pH, osmolality (Osmol/kg), buffer capacity and ionic strength. The media are optimised to simulate human and canine physiological fluids in the stomach and small intestines more closely than forerunners in the case of human media and to simulate the canine fluids for the first time.

The terms "FaSSGF *human"* and "FaSSIF *human",* "FaSSGF *canine"* and "FaSSIF *canine"* are narrower definitions than used in the prior art for FaSSGF and FaSSIF (without suffix). Prior art FaSSGF and FaSSIF are biorelevant media which are not standardised, for example optimised in that the key parameter, surface tension lies outside the biorelevant range of 25 mN/m to 50 mN/m, preferably between 30mN/m and 45mN/m, more preferably between 28mN/m and 45mN/m and more preferably between 30mN/m and 42mN/m.

"Biorelevant compositions" in the context of this specification are "instant" versions of homogeneous surfactant and optionally cosurfactant mixtures for reconsituting standardised "biorelevant media" *in situ.* Exemplary compositions may be homogeneous solid compositions for example powders, granules, pellets, tablets. Exemplary compositions may also be homogeneous liquid compositions for example aqueous concentrates comprising5 % to 60 % by weight of the surfactant mixtures.

"Standardised biorelevant media" in this specification describe aqueous biorelevant media which are optimised in terms of their physicochemical properties for example, pH, buffer capacity, osmolality, and in particular surface tension within the range defined by selections of bile salt and surfactants simulating fasted state conditions in the stomach and the small intestine. "Standardised biorelevant media" may be prepared, for example from dissolving or dispersing from scratch accurately weighed amounts of each of the components in aqueous media; or for example, by reconstituting "biorelevant compositions" using defined amounts within the range for preparing FaSSGF *human,* FaSSIF *human,* FaSSGF *canine* and FaSSIF *canine,* comprising a selection of surfactants and optionally cosurfactants. "Standardized biorelevant media" aim to target a surface tension or surface tension range within the overall range of between 25 mN/m and 50mN/m, preferably in the range of 30mN/m to 45mN/m, more preferably between 28mN/m and 45mN/m, and more preferably between 30mN/m and 42mN/m.

The targeted value/s is/are specific for the combination of species/location in the GI tract/prandial state to be simulated and which should be reproduced consistently each time the media is prepared within the definition for "standardised biorelevant media".

The singular and the plural terms in this specification are interchangeable.

### Prior Art

The prior art in the development of biorelevant intestinal media generally reports on solubility and dissolution assessments on poorly soluble compounds and their dosage forms using for example FaSSIF and FeSSIF for *in vitro-in vivo* correlation and prediction of *in vivo* drug release (Dressman et. al., Dissolution testing as a prognostic tool for oral drug absorption: immediate release dosage forms. Pharm. Res. 15:11-22 (1998). IVISIV *(In vitro-in silico-in vivo)* modelling and simulation has recently evolved relying on *in vitro* solubility and dissolution data input from biorelevant media (Shono et. al., Prediction of food effects on the absorption of celecoxib based on biorelevant dissolution testing coupled with physiologically based pharmacokinetic modelling. Europ. Journal Pharm. and Biopharm. 73 107-114 (2009)). For closer simulation of physiological fluids and to provide better prediction of drug release, FaSSIF V2 and FeSSIF V2 have been suggested (Jantratid et. al., Dissolution Media Simulating Conditions in the Proximal Human Gastrointestinal Tract: An Update. Pharmaceutical Research, Vol. 25, No. 7, (2008)).

Physicochemical factors such as molar concentrations and mole ratios of the surfactants, pH, osmolality, viscosity and surface tension in human intestinal aspirates which can affect the dissolution characteristics and absorption of poorly soluble compound are reviewed in the prior art (Kleberg, Journal of Pharmacy & Pharmacology 62:1656-1668 (2010)). However, no particular factor other than food effects and the levels, for example, molar concentrations of bile salts and phospholipids in the GI tract were emphasized for providing simulated physiological fluids for *in vitro* solubility and dissolution testing. No particular component (s) responsible for targeting surface tension was identified in preparing FaSSGF or FaSSIF. In particular, components which can be used to target surface tension and may affect the aggregation state, for example organisation of the mixed micelles in simulated media, have not been disclosed for preparing either FaSSGF or FaSSIF for testing (Fotaki and Vertzoni. The Open Drug Delivery Journal, 2010, 4, 2-13).

As far as it is known, there is no disclosure in the prior art relating to FaSSGF and FaSSIF *human and canine* media (including other mammalian species) targeting and characterised by the surface tension parameter as described in this invention.

WO 2007/054342 discloses solid dissolution compositions and method of preparing human biorelevant media comprising both FaSSIF and FeSSIF. The solid compositions describe bile salt and phospholipid complexes consisting of bile salt and phospholipid in the molar ratio of 1:1 to 20:1. The phospholipid may be a diacyl phospholipid or a monoacyl phospholipid and mixtures of diacyl and monoacyl phospholipids. There is no disclosure on FaSSGF nor the components and their levels which can affect and target surface tension in FaSSGF or FaSSIF for humans. Nor does the citation specify a method of targeting surface tension for FaSSGF and FaSSIF.

WO 2008/040799 describes instant forms of biorelevant media comprising bile salt and phospholipids in the ratio 1:1 and 10:1 and optionally breakdown products of triglyceride digestion such as a mono glyceride and a fatty acid in a ratio of 1:10 to 6:1 in relation to the bile salt for preparing only Fed State Simulated Intestinal Fluid (FeSSIF). Components which can be used to manipulate surface tension and target the desired range in FaSSIF were not identified and the citation is outside the scope of this invention.

Above all, neither of these two citations discloses standardised biorelevant media according to the definition in this specification, optimised and characterised by physicochemical properties in particular, but not limited to surface tension within the range defined by selections of bile salt and surfactants simulating fasted state conditions in the stomach and the small intestine of human and other mammalian species. Further, the citations fail to teach separate biorelevant media for animals typically used in preclinical evaluations of pharmaceutical products from those media to be used to evaluate different formulations for human medicine and for selection of the optimal formulation to be used in clinical trials, to aid in de-risking bioequivalence studies prior to or after marketing authorization has been obtained and thus to streamline pharmaceutical development of drug products.

### Object of the Invention

It is an object of the invention to provide standardised FaSSGF *human* and FaSSIF *human* and in addition standardised biorelevant media for pre-clinical animal species including but not limited to dog, mouse, rat, rabbit, guinea pig, monkey and pig, The optimised biorelevant media most closely and consistently simulate the desired mammalian stomach and intestinal fluids in the fasted state, which is known to vary among species. It is a further object of the invention to provide a method of preparing biorelevant media for better simulating fasted state conditions in the stomach and upper intestine of mammals. The biorelevant media may be characterised by physicochemical properties in particular a target range for surface tension for *in vitro* studies. The media are used for *in vitro* solubility, permeability, supersaturation, precipitation, release and dissolution of poorly water soluble compounds and their dosage forms. Non-human simulated media can also be used to evaluate *in vitro* solubility and release profiles of drugs for veterinary use. For example, canine media can be used for veterinary *in vitro* assessments in dogs, thereby minimizing the number of *in vivo* studies required to optimize the formulation for commercial purposes and reduces the risks of relying heavily on *in vivo* bioequivalence studies

### Summary

This invention discloses standardised, for example optimised biorelevant media composed of at least 40 mole % of at least one bile salt and the remaining mole % made up of a combination of at least two named surfactants and optionally cosurfactants, simulating fasted state conditions in the stomach and the upper small intestine of a given mammalian species including but not limited to humans and dogs characterised by surface tension in the range between 25 mN/m and 50 mN/m, preferably between 30mN/m and 45mN/m, more preferably between 28mN/m and 45mN/m and more preferably between 30mN/m and 42 mN/m.

The invention describes standardised biorelevant media according to claims 1 to 10 comprising selections of named surfactant and optional cosurfactant mixtures for targeting in particular surface tension in the range between 25 mN/m and 50 mN/m.

Biorelevant compositions for reconstitution of fasted state standardised biorelevant media are disclosed in claims 11, 12, 13 and 14, wherein the biorelevant media is characterised by a surface tension parameter, targeting surface tension reproducibly between 25 mN/m and 50 mN/m for *in vitro* solubility and dissolution assessments, comparison of *in vitro* with *in vivo* results and paves the way for *in vitro - in silico - in vivo* (IVISIV) modelling and simulation.

In one exemplary aspect the invention describes a method for preparing the homogeneous *solid biorelevant* compositions of claim 13 by solvent removal in accordance with claim 15, or for providing homogeneous *liquid* biorelevant compositions, for example aqueous concentrates of claim 14 without solvent removal according to claim 16.

In another aspect a method for instantly preparing standardized biorelevant media for example, FaSSIF *human* and *canine,* and FaSSGF *human* and *canine* by adding aqueous media comprising buffers and osmotic regulators to defined amounts of homogeneous solid or liquid compositions is described in claims 17 and 18. Disclosed for the first time are separate FaSSGF *canine* and FaSSIF *canine* media which can be extended for other mammalian species and used for biorelevant *in vitro* solubility and dissolution tests in preference to FaSSGF and FaSSIF *human* media designed to simulate human physiological conditions.

In a further exemplary aspect the standardized biorelevant media may be prepared by dissolving or dispersing individually weighed amounts of the components from scratch in aqueous medium according to claim 19.

The biorelevant media described in this invention can be used for example, for solubility testing, dissolution testing, drug release assessments, IVIVC, *in silico* modelling and simulation, drug supersaturation, drug precipitation, performance of enhanced formulations and drug permeability studies according to claim 20.

Further uses of non human biorelevant media are described in detail and include assessing *in vitro* dissolution and release profiles for formulation assessment of pre-clinical new chemical entities and bridging, to prediction of pharmacokinetic results in humans. Non human simulated media can also be used to evaluate *in vitro* solubility and release profiles of drugs for veterinary use. For example canine media can be used for veterinary *in vitro* assessments, thereby minimizing the number of *in vivo* studies required to optimize a pharmaceutical product for commercialization.

### Detailed Description

This invention describes standardised biorelevant media characterised by a surface tension parameter for *in vitro* dissolution testing using for example, FaSSGF *human,* FaSSIF *human,* FaSSGF *canine* and FaSSIF *canine* as highly valuable tools in pre-clinical development, formulation optimization, de-risking bioequivalence bridging studies, and in modelling and simulation. The media may be prepared from scratch for example, from dissolving or dispersing individual components in aqueous media.

The invention further describes biorelevant compositions which may be solid or liquid compositions using aqueous medium for reconstituting fasted state biorelevant media targeting surface tension in the range consistently between 25 mN/m and 50 mN/m.

The biorelevant compositions and the biorelevant media, characterised by surface tension between 25mN/n and 50mN/m comprise, for example, consist of
(i) selections from surfactants occurring in the gastro intestinal tract of mammals including bile salts, diacyl phospholipids, monoacyl phospholipids, fatty acids or monovalent salts of fatty acids,
(ii) based on the total amount of surfactants selected from the group in (i), between 0% and 10 mole % of co-surfactants, for example between 0.001% and 10 mole %, naturally occurring in the gastro intestinal tract of mammals
including cholesterol and/or their esters, mono and/or diglycerides, triglycerides and decomposition products of phospholipids other than fatty acids.

In the embodiments below, the mole concentrations (mole %) of each surfactant and optional cosurfactant, total mole % of all the surfactants (in aqueous concentrates), and the mole ratios between the surfactants are separately provided for human and canine biorelevant compositions. Standardised biorelevant media characterised by the surface tension parameter, comprising defined amounts (mole %) of the biorelevant composition are also provided separately for human and canine. Modifications of the embodiments can be applied to provide standardised biorelevant media adapted for other animal species within the overall surface tension range of 25mN/m to 50mN/m.

### Human biorelevant composition and media, ratios and ranges

Exemplary biorelevant compositions and standardised biorelevant media characterised by surface tension between 25mN/n and 50mN/m for simulating fasted state conditions in humans comprise, for example, consist of:
(a) 40 mole % to 95 mole % of at least one bile salt, 0.1 mole % to 40 mole % of at least one diacyl phospholipid, 0. 1 mole % to 40 mole % of at least one fatty acid or monovalent salt of fatty acid, or
(b) 40 mole % to 95 mole % of at least one bile salt, 0.1 mole % to 40 mole % of at least one monoacyl phospholipid; 0.1 mole % to 40 mole % of at least one fatty acid or monovalent salt of fatty acid, or
(c) 40 mole % to 95 mole % of at least one bile salt, 0.1 mole % to 40 mole % of at least one diacyl phospholipid, 0.1 mole % to 40 mole % of at least one monoacyl phospholipid; 0.1 mole % to 40 mole % of at least one fatty acid or monovalent salt of fatty acid, or
(d) 40 mole % to 95 mole % of at least one bile salt, 0.1 mole % to 40 mole % of at least one diacyl phospholipid and 0. 1 mole % to 40 mole % of at least monoacyl phospholipid
(e) mixture of (a) or (b) or (c) or (d) and between 0 mole % and 10 mole % cosurfactants for example cholesterol between 0.001 mole % and 10 mole %.

The ranges for the mole concentration (mole %) of each of the surfactants and optional co-surfactants in the human biorelevant compositions and resulting standardised biorelevant media are;
- at least one bile salt: between 40 and 95 mole % (at least 40 mole % and between 40 mole % and 50 mole %), (at least 50 mole % and between 50 mole % and 60 mole %), (at least 60 mole % and between 60 mole % and 70 mole %), preferably (at least 70 mole % and between 70 mole % and 80 mole %), more preferably (at least 80 mole % and between 80 mole % and 90 mole %) and most preferably (at least 90 mole % and between 90 mole % and 95 mole %).
- at least one diacyl phospholipid: between 0.1% and 40 mole % (preferred between 0.5 % and 30 %, between 1 % and 20 %, more preferred between 2% and 20%; more preferred between 1 % and 15 %;
- at least one monoacyl phospholipid: between 0.1% and 40 mole % (preferred between 0.5 % and 30 %, between 1 % and 20 %, more preferred between 2% and 20%; more preferred between 1 % and 15 %,);
- at least one fatty acid or monovalent salt of fatty acid: between 0.1% and 40 mole % (preferred between 0.5 % and 30 %, between 1 % and 20 %, more preferred between 2% and 20%; more preferred between 1 % and 15 %,);
- cholesterol: between 0% and 10 mole %, (preferably between 0.001% and 10 mole %, preferred between 0.01% and 7.5%, more preferred between 0.01 % and 5 %, more preferred between 0.01% and 1%).

The ranges for the mole ratios between the surfactants selected in the human biorelevant compositions and resulting standardised biorelevant media are:
- The mole ratio of bile salts to the rest of the surfactants comprising diacyl phospholipids, monoacyl phospholipids, fatty acids or monovalent salts of fatty acids, optionally cholesterol in the mixture is 1:2 to 20:1.
- The mole ratio of bile salts to the diacyl phospholipids and monoacyl phospholipids is 1:1 to 20:1, preferably 4:1 to 15:1, more preferably between 10:1 and 15:1.
- The mole ratio of diacyl phospholipids to fatty acids or monovalent salts of fatty acid in the mixture in (a) is 1:20 to 20:1, preferably about 1:5 to 5:1, more preferably 1:2 to 2:1.
- The mole ratio of monoacyl phospholipids to fatty acids or monovalent salts of fatty acids in the mixture in (b) is 1:20 to 20:1, preferably about 1:5 to 5:1,more preferably 1:2 to 2:1.
- The mole ratio of monoacyl phospholipids and diacyl phospholipids to fatty acids or monovalent salts of fatty acids in the mixture (c) is 1:20 to 20:1, preferably about 1:5 to 5:1, more preferably 1:2 to 2:1.
- The mole ratio of diacyl phospholipids to monoacyl phospholipids in the mixture (d) is 1:30 to 2:1, preferably about 1:25 to 1:1, more preferably 1:20 to 1:2.

### Canine biorelevant composition and media, ratios and ranges

Exemplary biorelevant compositions and standardised media characterised by surface tension between 25mN/n and 50mN/m for simulating fasted state conditions in dogs comprise, for example, consist of:
(a) 40 mole % to 95 mole % of at least one bile salt, 0.1 mole % to 40 mole % of at least one diacyl phospholipid, 0. 1 mole % to 40 mole % of at least one fatty acid or monovalent salt of fatty acid, or
(b) 40 mole % to 95 mole % of at least one bile salt, 0.1 mole % to 40 mole % of at least one monoacyl phospholipid; 0.1 mole % to 40 mole % of at least one fatty acid or monovalent salt of fatty acid, or
(c) 40 mole % to 95 mole % of at least one bile salt, 0.1 mole % to 40 mole % of at least one diacyl phospholipid, 0.1 mole % to 40 mole % of at least one monoacyl phospholipid; 0.1 mole % to 40 mole % of at least one fatty acid or monovalent salt of fatty acid, or
(d) 40 mole % to 95 mole % of at least one bile salt, 0.1 mole % to 40 mole % of at least one diacyl phospholipid and 0. 1 mole % to 40 mole % of at least monoacyl phospholipid
(e) mixture of (a) or (b) or (c) or (d) and between 0 mole % and 10 mole % cosurfactants for example cholesterol between 0.001 mole % and 10 mole %.

The ranges for the mole concentration (mole %) of each of the surfactants and optional co-surfactants selected in the canine biorelevant compositions and resulting standardised biorelevant media are;
- at least one bile salt: between 40 and 95 mole % ((at least 40 mole % and between 40 mole % and 50 mole %), (at least 50 mole % and between 50 mole % and 60 mole %), (at least 60 mole % and between 60 mole % and 70 mole %), preferably (at least 70 mole % and between 70 mole % and 80 mole %), more preferably (at least 80 mole % and between 80 mole % and 90 mole %) and most preferably (at least 90 mole % and between 90 mole % and 95 mole %);
- at least one diacyl phospholipid: between 0.1% and 40 mole % (preferred between 0.5 % and 30 %, between 1 % and 20 %, more preferred between 2% and 20%; more preferred between 1 % and 15 %),
- at least one monoacyl phospholipid: between 0.1% and 40 mole % (preferred between 0.5 % and 30 %, between 1 % and 20 %, more preferred between 2% and 20%; more preferred between 1 % and 15 %,);
- at least one fatty acid or monovalent salt of fatty acid: between 0.1% and 40 mole % (preferred between 0.5 % and 30 %, between 1 % and 20 %, more preferred between 2% and 20%; more preferred between 1 % and 15 %,);
- cholesterol: between 0% and 10 mole %, (preferably between 0.001% and 10 mole %, preferred between 0.01% and 7.5%, more preferred between 0.01 % and 5 %, more preferred between 0.01% and 1%).

The ranges for the mole ratios between the surfactants selected in the canine biorelevant compositions and resulting standardised biorelevant media are:
- The mole ratio of bile salts to the rest of the surfactants comprising diacyl phospholipids, monoacyl phospholipids, fatty acids or monovalent salts of fatty acids, optionally cholesterol in the mixture is 1:2 to 20:1.
- The mole ratio of bile salts to the diacyl phospholipids and monoacyl phospholipids is 1:1 to 20:1, preferably 4:1 to 15:1, more preferably between 10:1 and 15:1.
- The mole ratio of diacyl phospholipids to fatty acids or monovalent salts of fatty acid in the mixture in (a) is 1:20 to 20:1, preferably about 1:5 to 5:1, more preferably 1:2 to 2:1.
- The mole ratio of monoacyl phospholipids to fatty acids or monovalent salts of fatty acids in the mixture in (b) is 1:20 to 20:1, preferably about 1:5 to 5:1,more preferably 1:2 to 2:1.
- The mole ratio of monoacyl phospholipids and diacyl phospholipids to fatty acids or monovalent salts of fatty acids in the mixture (c) is 1:20 to 20:1, preferably about 1:5 to 5:1, more preferably 1:2 to 2:1.
- The mole ratio of diacyl phospholipids to monoacyl phospholipids in the mixture (d) is 1:30 to 2:1, preferably about 1:25 to 1:1, more preferably 1:20 to 1:2.

FaSSIF *human* and FaSSGF *human* and FaSSIF *canine* and FaSSGIF *canine* for targeting surface tension reproducibly between 25 mN/m and 50 mN/m and preferably in the range between 30mN/m and 45mN/m comprise selections of surfactant mixtures defined by content for example (a) mole per cent of each named surfactant and optional cosurfactant (b) the mole ratios of the surfactants in relation to each other (c) the total amount (mole %) of all the surfactants and optional cosurfactants in the media, optimized to match as closely as possible the fluids in the stomach and in the target location of the upper small intestine of the given mammal. For other mammalian species, the optimal surface tension for a given location in the intestinal tract the species of interest will need to be taken into consideration to determine the target surface tension value/s (within the overall range of 25-50 mN/m).

The prior art has neither considered targeting surface tension of the biorelevant media, nor identified a range for *in vitro* testing.

There is no disclosure in the prior art relating to homogeneous biorelevant compositions or biorelevant media comprising defined amounts of said compositions for preparing for example FaSSIF *human* and FaSSGF *human* FaSSIF *canine* and FaSSGF *canine* media characterised by surface tension in the range between 25 mN/m and 50 mN/m and preferably between 30 mN/m and 45 mN/m, more preferably between 28mN/m and 45mN/m and more preferably between 30 mN/m and 42mN/m.

Biorelevant media which consist of binary mixtures of only bile salts and only diacyl phospholipids form mixed micelles in aqueous medium and may not provide solutions or dispersions with surface tension reproducibly, optimised between 25 mN/m and 50 mN/m.

Prior art FaSSIF and FaSSIFv2 do not provide in particular the selection of surfactants and cosurfactants disclosed herein. The surface tension in the comparative examples shown for prior art FaSSIF is about 52 mN/m (comparative example 9) and for FaSSIF v2 about 54 mN/m (comparative example 8), which are outside the range for standardised FaSSGF *human* and FaSSIF *human* media claimed in this invention, for example reproducibly between 25 mN/m to 50 mN/m, preferably in the range between 30nM/m and 45nM/m.

According to the present invention, FaSSGF *human* and FaSSIF *human* are prepared either for example, by dissolving or dispersing separately weighed amounts of surfactants and optional cosurfactants from scratch in aqueous medium; or alternatively dissolving or dispersing defined amounts of the solid composition for example powders, or diluting the liquid composition for example aqueous concentrates, in the aqueous medium. Aqueous media comprise components selected from but not limited to buffers, osmotic components, stabilizers, antioxidants, pH adjusters, antimicrobials, enzymes.

Preparing standardised biorelevant media from solid compositions for example powders, or liquid compositions, for example aqueous concentrates is more cost effective and has the advantage that the media, which have limited stability once prepared in the final form for use, need not be stored. They can be freshly made up instantly *in situ* as required in desired aqueous medium, with minimum inter-batch variation and weighing inaccuracies. By contrast, making up the media from scratch each time using individually weighed components is not the most cost and time efficient. Furthermore, separating the buffers and osmotic components from the homogeneous solid or liquid compositions confer greater flexibility for selecting and tailoring the media to the desired pH and osmotic pressure in the different locations in the GI tract. The biorelevant compositions ("instant" versions) of the biorelevant media are constituted with surfactants and optional cosurfactants to afford the possibility of combining them with buffers and osmotic agents appropriate to the species and the segment of the gastrointestinal tract to be simulated, as well as variations in physiological conditions at these locations, into consideration. As an illustration, it may be desirable to know how a drug product will perform in humans that are being treated with gastric acid blockers (e.g. proton pump inhibitors) *vis a vis* in humans with normal, low gastric pH. In such a case, the choice of diluent or aqueous medium will be different for the two situations, although the same biorelevant composition can be used as the starting point for the reconstitution of the biorelevant media. Similarly, in some pre-clinical studies, dogs are administered for example by iv injection, pentagastrin to stimulate gastric acid production while in other studies this is not done and the dogs will have a higher gastric pH. To forecast the *in vivo* outcome, buffers with appropriate pH, buffer strength and osmolarity can be used to reconstitute the biorelevant composition or to make up the standardized biorelevant media from scratch.

It can be shown that mixtures of diacyl and monoacyl phospholipids or diacyl phospholipids and fatty acids including monovalent salts can arrange as colloidal aggregates comprising for example non-vesicular micelles, vesicular aggregates and mixed micelles. The surfactant mixtures form heterogeneous populations which comprise various forms of vesicular and nonvesicular aggregates depending on the surfactant type; mole % of the surfactants individually and in total; ratios between the surfactants in the mixture. As a rule, smaller head groups in monoacyl phospholipids and fatty acids form micelles wherein the molecular arrangements provide hydrocarbon cores for solubilizing lipophilic drugs. In comparison, the larger head groups in diacyl phospholipids predispose towards vesicular aggregates Therefore micellar and vesicular aggregates may provide different solubilization capacity for drugs.

When either monoacyl phospholipids or fatty acids are present separately or together in the mixture comprising bile salts and diacyl phospholipids, the smaller head groups in monoacyl phospholipids and fatty acids may alter the aggregation state of the mixed micelle population formed with only bile salt and diacyl phospholipids. Micelles and mixed micelles comprising mixtures of bile salts and diacyl phospholipids can have variable surface tension which can be less than 25 mN/m or above 50mN/m depending on selection of the molar concentrations and mole ratios of the surfactants in the mixture. The surface tension of water alone is 72.8 mN/m measured at room temperature. Buffers do not significantly affect the surface tension of water.

The observation that the surface tension of upper gastrointestinal fluids lies within a band suggest that for more closely simulating physiological fluids in the fasted state, other biorelevant surfactants and cosurfactants than just bile salts and diacyl phospholipids and, in particular, their mole concentration and mole ratios should be taken into account. This invention describes biorelevant compositions and a method for preparing standardised biorelevant media simulating fasted state conditions characterised by reproducible surface tension between 25mN/m and 50mN/m within the meaning of the definition for standardised. The biorelevant compositions and media claimed in this invention comprise a selection of physiological surfactants and optional cosurfactants other than just bile salts and diacyl phospholipids in the mixture. For example the surfactant components for targeting surface tension broadly between 25mN/m and 50mN/m may be selected from bile salts, diacyl phospholipids, monoacyl phospholipids, fatty acids and their monovalent salts, and optionally cosurfactants occurring naturally in the gastrointestinal tract.

Without being bound to the explanation, surface tension may result from the interplay between the surfactants in the mixture resulting in colloidal aggregates with various molecular arrangements in the bulk liquid media, for example in the form of micelles, mixed micelles and vesicles. Further, the surfactant mixture may result in some surfactant species not being included in colloidal aggregates but existing as monomers below the critical micelle concentration (CMC). Surface tension is exerted at the air/liquid interface or liquid/solid interface and may express the overall aggregation state of the surfactant mixtures. Thus surface tension of the biorelevant media may be a useful physicochemical parameter for characterising and standardising the physicochemical properties of heterogeneous surfactant mixtures. Further, surface tension is a desirable property because lowering the surface tension leads to an increase in contact ("wetting") between the biorelevant media and the surface of poorly soluble drug particles or drug products thereby facilitating dissolution.

Pre-clinical studies of oral dosage forms are generally carried out in dogs. Other pre-clinical animal species include but are not limited to mouse, rat, rabbit, guinea pig, monkey and pig. Biorelevant media employed up to- date in early drug development studies in canine models for *in vitro -in vivo* correlation and prediction, for example FaSSGF, FaSSIF, FaSSIFv2, etc. had actually been designed for human studies. There are differences in the composition of gastric and intestinal fluids in humans and canine species for example in pH and composition of bile salts and phospholipids in the fasted state. Therefore, it makes sense to provide separate canine biorelevant media for in vitro tests of API and formulation performance. It is to be understood that this invention describes fasted state biorelevant media simulating gastric and intestinal fluids across different mammalian species defined by surface tension within the range of 25 mN/m to 50 mN/m for the specific purpose of *in vitro* solubility, dissolution and permeability assessments and correlations with *in vivo* data in a given mammal.

Disclosed for the first time are canine_biorelevant media characterised by physicochemical parameters which include surface tension that simulate canine fasted state gastric conditions herein known as FaSSGF *canine* and canine fasted state simulated intestinal fluid herein known as FaSSIF *canine.* Dissolution and solubility data obtained in canine biorelevant media provide better correlation to canine *in vivo* PK data compared to using human biorelevant media.

*In vitro* testing in canine biorelevant media and establishing IVIV correlation in canine can facilitate approval of veterinary products and for comparing *in vitro* release data in canine media with *in vitro* data using human media. It can also facilitate rational selection of the most appropriate pre-clinical test species without involving large number of *ad hoc in vivo* trials in different species.

Exemplary FaSSGF *canine* and FaSSIF *canine* comprise at least one bile salt which may be for example Taurocholate, or a mixture of Taurocholate and Taurodeoxycholate, for example approximately in equal proportions. Mixtures of the bile salts identified herein combined with at least two named surfactant from a group and optional cosurfactant(s) provide standardised biorelevant media with surface tension in the range between 25mN/m and 59mN/m, preferably between 30 mN/m and 45 mN/m, between 28mN/m and 45mN/m and more preferably between 30 mN/m and 42mN/m.

Further, targeting surface tension may be a viable method to optimise and standardise biorelevant mammalian fasted state gastric and intestinal media which better simulate physiological fluids. Surface tension can be targeted and standardised between 25 mN/m and 50 mN/m, preferably for example between 30 mN/m and 45 mN/m by varying the contents of the mixture and the amount of surfactants.

According to the prior art, the solubility of the poorly soluble base ketoconazole in prior art FaSSIF (Söderlind) is 26 µg/ml. In dog intestinal aspirates (Kalantzi) the solubility is between 30 and 160 µg/ml. In comparison the solubility in FaSSIF *canine* is 84.2 µg/ml and therefore within the range found in actual dog aspirates.

The solubility of the poorly soluble base dipyridamole in prior FaSSIF (Söderlind) is 19 µg/ml. In canine intestinal aspirates (Kalantzi) the solubility is between 25 and 95 µg/ml. In comparison, the solubility in FaSSIF *canine* is 75.0 µg/ml and within the range found in actual dog aspirates.

Surprisingly, whereas monoacyl phospholipids and fatty acids or monovalent salts of fatty acids; diacyl phospholipids and fatty acids or monovalent salts of fatty acids may decrease surface tension, cholesterol may increase surface tension. Cholesterol may be included in the surfactant mixture in amounts up to 10 mole %, for example between 0.001 mole % and 10 mole %. Including cholesterol in FaSSIF *human* or FaSSGF *human* for simulating physiological fluids may provide closer simulation for testing solubility or dissolution of lipophilic drugs and formulations. Whether or not cholesterol is included in biorelevant media to simulate physiological fluids in the fasted state is optional and depends on the drug to be assessed. Cosurfactants for example cholesterol and its esters and amounts between 0% and 10mole %, or for example between 0.001% and 10 mole % may be included in the fasted state biorelevant composition and the media thereof to provide a surface tension between 25mN/m and 50mN/m. Further, the cosurfactants may be naturally occurring in the gastro intestinal tract of mammals, for example cholesterol or their esters, monoglycerides, diglycerides, triglycerides, decomposition products of phospholipids other than fatty acids and mixtures thereof.

In one embodiment standardised biorelevant media for simulating fasted state stomach and fasted state upper small intestinal fluids of mammalian species characterised by surface tension between 25 mN/m and 50 mN/m, preferably in the range of 30mN/m to 45mN/mcomprise or preferably consist of surfactants selected from the group consisting of:
(i) surfactants occurring in the gastro intestinal tract of mammals including bile salts, diacyl phospholipids, monoacyl phospholipids, fatty acids or monovalent salts of fatty acids,
(ii) between 0% and 10 mole % cosurfactants occurring in the gastro intestinal tract of mammals including cholesterol or their esters, monoglycerides, diglycerides, triglycerides, decomposition products of phospholipids other than fatty acids and mixtures thereof.

Exemplary standardised biorelevant media characterised by surface tension between 25 mN/m and 50 mN/m for simulating fasted state conditions in human and mammalian species comprise or preferably consist of:
(a) 40 mole % to 95 mole % of at least one bile salt, 0.1 mole % to 40 mole % of at least one diacyl phospholipid, 0. 1 mole % to 40 mole % of at least one fatty acid or monovalent salt of fatty acid, or
(b) 40 mole % to 95 mole % of at least one bile salt, 0.1 mole % to 40 mole % of at least one monoacyl phospholipid; 0.1 mole % to 40 mole % of at least one fatty acid or monovalent salt of fatty acid, or
(c) 40 mole % to 95 mole % of at least one bile salt, 0.1 mole % to 40 mole % of at least one diacyl phospholipid, 0.1 mole % to 40 mole % of at least one monoacyl phospholipid; 0.1 mole % to 40 mole % of at least one fatty acid or monovalent salt of fatty acid, or
(d) 40 mole % to 95 mole % of at least one bile salt, 0.1 mole % to 40 mole % of at least one diacyl phospholipid and 0. 1 mole % to 40 mole % of at least monoacyl phospholipid
(e) mixture of (a) or (b) or (c) or (d) and between 0% and 10 mole % cosurfactants, for example cholesterol between 0.001 mole % and 10 mole %.

The surfactants may be added separately to aqueous media to prepare the biorelevant media or they may be in a biorelevant composition for example a homogeneous solid composition or liquid composition for example aqueous concentrate for reconstitution with aqueous medium.

In one embodiment the biorelevant media are obtained from the powder biorelevant composition by adding aqueous medium comprising components selected from water, buffer, osmotic components, enzymes for example pepsin or pancreatic enzymes.

In another embodiment the biorelevant media are obtained by diluting the liquid biorelevant composition for example aqueous concentrates with aqueous medium comprising components selected from water, buffer, pH adjusters, osmotic components, stabilizers, antioxidants, antimicrobials, enzymes for example pepsin or pancreatic enzymes.

In one embodiment FaSSGF *human* comprising between 0.01 mmol and 5 mmol, preferably between 0.01 mmol and 1 mmol of surfactants and optional cosurfactants; or FaSSIF *human* comprising between 2mmol and 20mmol (preferably 2mmol to 6 mmol, more preferably 3mmol to 5 mmol of surfactants and optional cosurfactants, are obtained by adding aqueous medium containing components selected from buffers, osmotic components, stabilizers, antioxidants, pH adjusters, antimicrobial, enzymes for example pepsin to solid or liquid biorelevant compositions.

In one embodiment FaSSGF *canine* comprising between 0.01 mmol and 5 mmol, preferably between 0.1 mmol and 1 mmol; or FaSSIF *canine* comprising between 2mmol and 20 mmol (preferably 10mmol to 15 mmol, more preferably 12mmol to 14 mmol) of surfactants and optional cosurfactants are obtained by adding aqueous medium containing components selected from buffers and osmotic regulators, stabilizers, antioxidants, pH adjusters, antimicrobial, enzymes for example pepsin, to solid or liquid biorelevant compositions.

The lower surfactant concentrations for preparing FaSSGF *human and canine* compared to FaSSIF *human and canine* reflect the small amounts found in the stomach due to reflux of intestinal contents.

In one embodiment FaSSIF *human* or FaSSIF *canine* optionally comprise between 0.001 mole % and 10 mole % cosurfactant, for example cholesterol.

### FaSSGF human and FaSSIF human pH

In one embodiment the pH of FaSSGF *human* is between pH 1 and 3, for example about pH1.6.

In one embodiment the pH of FaSSIF *human* is between 5 and 8, for example about pH 6.8.

### FaSSGF canine and FaSSIF canine pH

Furthermore in separate embodiments FaSSGF *canine* at pH 1-3, for example pH 1.5 and FaSSGF *canine* at pH 5-8, for example pH 6.5 simulate *in vitro* the physiological gastric fluids of dog which are treated with and without pentagastrin respectively.

In one embodiment FaSSGF *canine* is at for example pH 1.5 to test the solubility and dissolution of poorly soluble drugs, particularly acidic drugs, or precipitation of soluble salt forms in stomach juices at acid pH 1.5 or for example at pH 6.5 to test the solubility and dissolution of poorly soluble drugs particularly acid drugs, or precipitation of soluble salt forms in stomach juices at acid pH 6.5 to mimic effects of, for example antacids, H2 antagonists and inhibitors which suppress acid production in the stomach.

In one embodiment the pH of FaSSIF *canine* is between pH 6 to 9, for example pH 7.5.

In one embodiment optionally 0.1 mg/mL to 1 mg/mL of pepsin may be added to FaSSGF *human* or FaSSGF *canine.*

### FaSSIF human osmolarity and buffer capacity

In one embodiment the osmolarity of FaSSIF *human* is in the range between 175mOsm/kg and 280mOsm/kg, preferably between 130 mOsm/kg and 225mOsm/kg - for example about 200mOsm/kg.

In one embodiment the buffer capacity of FaSSIF *human* is in the range between 2.5 mmol/l/ΔpH and 6.0mmol/l/ΔpH, preferably between 3 mmol/l/ΔpH and 5.8mmol/l/ΔpH for example about 5.6mmol/l/ΔpH.

### FaSSIF canine osmolarity and buffer capacity

In one embodiment the osmolarity of FaSSIF*canine* is in the range between 25 mOsm/kg and 600 mOsm/kg, preferably between 50 mOsm/kg and 300mOsm/kg, more preferably between 100mOsm/kg and 250mOsm/kg, for example 180mOsm/kg.

In one embodiment the buffer capacity of FaSSIF *canine* is in the range between 1.0 mmol/l/ΔpH and 50mmol/l/ΔpH, preferably between 2 mmol/l/ΔpH and 30mmol/l/ΔpH, more preferably between 5mOsm/kg and 15 mOsm/kg for example about 10mmol/l/ΔpH.

### FaSSGF human osmolarity and buffer capacity

In one embodiment the osmolarity of FaSSGF*human* is in the range between 10mOsm/kg and 400 mOsm/kg, preferably between 25 mOsm/kg and 300mOsm/kg, more preferably between 50mOsm/kg and 200mOsm/kg, for example about 120mOsm/kg.

In one embodiment the buffer capacity of FaSSGF *human* is in the range between 0 mmol/l/ΔpH and 50mmol/l/ΔpH, preferably between 0 mmol/l/ΔpH and 30mmol/l/ΔpH, more preferably between 0mOsm/kg and 10mOsm/kg.

### FaSSGF canine osmolarity and buffer capacity

In one embodiment the osmolarity of FaSSGF*canine* is in the range between 10 mOsm/kg and 400 mOsm/kg, preferably between 25 mOsm/kg and 200mOsm/kg, more preferably between 50mOsm/kg and 150mOsm/kg, for example about 100mOsm/kg.

In one embodiment the buffer capacity of FaSSGF *canine* is in the range between 1 mmol/l/ΔpH and 50mmol/l/ΔpH, preferably between 2 mmol/l/ΔpH and 30mmol/l/ΔpH, more preferably between 5mOsm/kg and 15 mOsm/kg for example about 10mmol/l/ΔpH.

In one embodiment the biorelevant compositions may be solid or liquid compositions for preparing standardised biorelevant media simulating fasted state stomach and fasted state upper small intestinal fluids of mammalian species characterised by surface tension between 25 mN/m and 50 mN/m preferably in the range of 30mN/m to 45mN/m comprising, or preferably consist of members selected from the group of surfactants consisting of:
(i) surfactants naturally occurring in the gastro intestinal tract of mammals including bile salts, diacyl phospholipids, monoacyl phospholipids, fatty acids or monovalent salts of fatty acids,
(ii) between 0% and 10 mole % cosurfactants naturally occurring in the gastro intestinal tract of mammals including cholesterol or their esters, mono, diglycerides, triglycerides, decomposition products of phospholipids other than fatty acids and mixtures thereof.

In one embodiment the solid or liquid biorelevant composition for preparing biorelevant media targeting surface tension in human and mammalian for example *canine* FaSSIF and FaSSGF characterised by surface tension between 25mN/m and 50mN/ preferably in the range of 30mN/m to 45mN/m comprise preferably consist of:
(a) 40 mole % to 95 mole % of at least one bile salt, 0.1 mole % to 40 mole % of at least one diacyl phospholipid, 0. 1 mole % to 40 mole % of at least one fatty acid or monovalent salt of fatty acid, or
(b) 40 mole % to 95 mole % of at least one bile salt, 0.1 mole % to 40 mole % of at least one monoacyl phospholipid; 0.1 mole % to 40 mole % of at least one fatty acid or monovalent salt of fatty acid, or
(c) 40 mole % to 95 mole % of at least one bile salt, 0.1 mole % to 40 mole % of at least one diacyl phospholipid, 0.1 mole % to 40 mole % of at least one monoacyl phospholipid; 0.1 mole % to 40 mole % of at least one fatty acid or monovalent salt of fatty acid, or
(d) 40 mole % to 95 mole % of at least one bile salt, 0.1 mole % to 40 mole % of at least one diacyl phospholipid and 0. 1 mole % to 40 mole % of at least monoacyl phospholipid
(e) mixture of (a) or (b) or (c) or (d) and between 0 mole % to 1 mole % cosurfactants for example cholesterol between 0 mole % and 10 mole %.

In one embodiment the method for preparing solid biorelevant compositions includes a step which comprises dissolving the surfactants and optionally cosurfactants in a solvent, water or mixtures thereof and eliminating the solvent, thereby providing a homogeneous solid composition wherein the moisture content is below 5 % by weight, preferably below 3 % by weight.

In one embodiment the dried solid composition is milled and screened or sieved to obtain a powder composition with mean particle diameter between 10 µm and 1000 µm preferably 50 µm to 500 µm; bulk density between 0.3 g/m³ and 0.7 g/cm; moisture content below 5 % by weight; granules; pellets with mean particle diameter 200 to 2000 µm; tablets; or capsules.

In an alternative embodiment the method for preparing liquid biorelevant compositions for example a homogeneous aqueous concentrate comprising between 5 % and 60 %, preferably 10 % to 40 % and most preferably 10 % to 30 % by weight of the surfactants and optional cosurfactants includes a step which comprises homogeneously dissolving or dispersing the surfactants and optionally cosurfactants in water at a temperature between 15°C and 60°C without a drying step to remove the water.

In another embodiment the biorelevant media are directly obtained by individually weighing and dissolving the surfactants and optionally cosurfactants separately, together or sequentially in the aqueous media comprising components selected from water, buffer, osmotic components, stabilizers, antioxidants, pH adjusters, antimicrobials.

The standardised biorelevant media according to the exemplary embodiments may be employed in for example, solubility testing, dissolution testing, drug release assessments, IVIVC, *in silico* modelling and simulation, drug supersaturation, drug precipitation, drug stability, performance of enhanced formulations and drug permeability studies.

**Bile Salts:** Sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glyco chenodeoxycholate, sodium cholylsarcosinate, sodium N-methyl taurocholate and their free acids. The cholates may be from natural, synthetic or semi-synthetic sources. If the cholate is natural, it should be preferably from porcine or TSE/BSE-free bovine sources.

Quality of bile salts: crude bile salts may also be used but do not offer the required reproducibility and consistency within the targeted values for the surface tension in the biorelevant media.

**Phospholipids** are selected from the group of phospholipids consisting of natural lecithins from egg yolk; soy bean; milk; sunflowers; oat comprising at least 20 % by weight, preferably 40 % by weight, and most preferably at least 95 % by weight phosphatidylcholine (PC) as the major component; and smaller amounts of phosphatidylethanolamine (PE); phosphatidylserine (PS); phosphatidic acid (PA), phosphatidylinositiol (PI); phosphatidylglycerol (PG) and mixtures thereof. In this specification, phospholipids also include cardiolipin, sphingomyelin, glycolipids.

**Diacyl phospholipids** consist of lecithin or phospholipids for example phosphatidylcholine (PC) listed above with twin fatty acid chains comprising between 14 and 24 carbon atoms attached to the head group.

**Monoacyl phospholipids** consist of lyso-lecithin or phospholipids for example phosphatidylcholine (PC) with only a single fatty acid chain. Monoacyl phospholipids are usually obtained by enzyme hydrolysis of phospholipid mixtures. Pure monoacyl phospholipids (above 95 %) for example monoacyl PC may be used. Alternatively mixtures of monoacyl phospholipids and diacyl phospholipids used in the biorelevant composition may be enzyme hydrolysed lecithin comprising at least 5 % monoacyl phospholipids. Mixtures comprising between 10 % and 95 % by weight monoacyl phospholipids after purification and fractionation may be used as the source for monoacyl phospholipids and taking into account the amount of diacyl phospholipids contained in the mixture. Preferably as the source for monoacyl phospholipids the mixture of monoacyl and diacyl phospholipids comprises between 70 % and 95 % by weight monoacyl phospholipids.

**Fatty acids** are selected from the group consisting of myristoleic acid, palmitoleic acid, sapienic acid, oleic acid, elaidic acid, vaccenic acid, linoleic acid, linoelaidic acid, linolenic acid, arachidonic acid, eicosapentaenoic acid, erucic acid, docosahexaenoic acid, caprylic acid, capric acid, lauric acid, myristic acid, palmitic acid, stearic acid, arachidic acid, behenic acid, lignoceric acid, cerotic acid comprising at least 60 % by weight, preferably 75 % by weight, and most preferably at least 85 % by weight of the desired fatty acid or mixtures of fatty acid.

**Monovalent salts of fatty acid** comprise the sodium or potassium salts of fatty acids from the list above comprising at least 98 % of the sodium or potassium salt form.

**Cholesterol**: cholesterol and cholesterol esters, comprising at least 80 % by weight, preferably 90 % by weight, and most preferably at least 95 % by weight of cholesterol or cholesterol ester.

### Molecular weight used for calculating molar concentrations and molar ratios.

| | |
|---|---|
| Sodium taurocholate | 538 |
| Monoacyl phospholipid | 505 |
| Diacyl phospholipid | 787 |
| Sodium oleate | 304 |
| Oleic acid | 282 |
| Cholesterol | 387 |

**Buffers and pH:** Exemplary buffer media to maintain pH at 1.5; 6.5 and 7.5 are described but not limited to Examples 11 to 13

**Osmotic components:** Exemplary osmotic components comprise but not limited to sodium chloride

### Method for preparing solid biorelevant compositions

The desired content and amount by weight of bile salts, diacyl phospholipids,monoacyl phospholipids, mixtures of diacyl and monoacyl phospholipids, fatty acids and monovalent salts, optionally cholesterol are all dissolved in volatile hydrophilic solvent or water and mixtures thereof, either separately and sequentially or in homogeneous admixture.

Preferred solvents are methanol, ethanol, tertiary butanol and combinations of hydrophilic solvents or dichloromethane on its own.

Mixtures of tertiary butanol and water preferably in equal amounts are particularly preferred.

After each surfactant is completely dissolved and a clear, white to yellowish solution is obtained, the solution is freeze-dried using a Christ Epsilon 2-4 LSC lyophilizer.

The surfactant solution is lyophilised to a solid composition with moisture content below 5 % by weight.

The solid composition is converted to a particulate composition by milling or grinding to a mean particle size range between 10 µm and 1000 µm.

After milling and screening the powder was ready to use for preparing fasted state biorelevant media using the desired molar concentration in the buffer solutions comprising osmotic components as shown in the examples.

### Physical characteristics of the powders

The mean particle size is in the range between 10 µm to 1000 µm.

The bulk density is between 0.3 g/cm³ to 0.7 g/cm^{3.}

The moisture content is below 5 % by weight preferably less than 3% by weight.

### Liquid biorelevant composition (aqueous concentrate)

The desired content and amount by weight of bile salts, diacyl phospholipids, monoacyl phospholipids, mixtures of diacyl and monoacyl phospholipids, fatty acids and monovalent salts, optionally cholesterol are all dissolved in or water, either separately and sequentially or in homogeneous admixture at a temperature between 15°C and 60°C. Stabilizers for example sodium azide, thiomersal, EDTA, tocopherols may be included in the liquid composition. After cooling to room temperature and filtration using for example a 0.22 µm filter, the liquid composition comprising between 5 % and 60 % by weight of the surfactants may be used to prepare the fasted state biorelevant media using the desired molar concentration in the buffer solutions comprising osmotic components.

### Physical Characteristics of the aqueous concentrate

Z verage particle size measured using PCS after diluting 1 % by weight of the liquid biorelevant composition to biorelevant medium: range 2 nm to 1000 nm.

Optical properties: Visually clear

### Method for measuring surface tension:

Surface tension measurements are carried out in a Kibron AquaPi tensiometer based on the DuNouy principle.

The instrument is pre-calibrated for a temperature of 20 °C. A correction factor is applied to any deviation.

The titanium rod is flamed to vaporize impurities before surface tension measurements are taken.

The sample cups are cleaned with ethanol and with purified water.

Recalibrations are carried out every time that the probe is changed or at least daily. Measurements are done in duplicate for calculating the average and the standard deviation. If the sample temperature deviates from 20°C the temperature correction factor is taken into consideration.

### Example 1:

### Preparation of a biorelevant powder composition for making FaSSIF human

Two grams of the solid biorelevant powder composition for the preparation of FaSSIF *human* biorelevant medium is prepared by dissolving 1.622g of sodium taurocholate (ST) in 10ml of purified water at room temperature using a magnetic stirrer. After the ST is completely dissolved and a clear solution is obtained 10ml of tert-butanol is added to the solution. In the next step 0.009g of diacyl phospholipid and 0.199g of monoacyl phospholipid is dissolved in the solution (alternatively the lipids are added in separate steps). After the lipid components are completely dissolved and a clear to slightly yellowish solution is obtained, 0.128g of sodium oleate is added to the solution. Finally after the sodium oleate is completely dissolved 0.043g of cholesterol is added to the solution. The final clear to slightly yellowish solution is transferred into a suitable container for freeze-drying.

### Example 2:

### Making FaSSIF human from the solid biorelevant composition shown in Example 1

| | | |
|---|---|---|
| Sodium taurocholate | 2.8 mmol | 1.518g/l |
| Diacylphospholipids | 0.035 mmol | 0.007 g/l |
| Monoacylphospholipids | 0.315mmol | 0.186 g/l |
| Sodium oleate | 0.35mmol | 0.120 g/l |
| Cholesterol | 0.1 mmol | 0.041 g/l |

| | | |
|---|---|---|
| pH | | 6.5 (maleate buffer) |
| **Surface tension** | | **34.7 mN/m** |

1.871g of the homogeneous powder composition from example 1 (comprising sodium taurocholate, diacyl phospholipids, monoacyl phospholipids sodium oleate and cholesterol) is dissolved in the maleate buffer comprising buffer and osmotic agents (example 13). The pH of the biorelevant medium is adjusted to pH 6.5.

Alternatively the equivalent amount by weight of a liquid composition for example an aqueous concentrate comprising 10% to 60% by weight of surfactants and optionally cosurfactants may be used in place of the powder composition.

Alternatively the components may be added separately.

### Example 3:

### Preparation of a biorelevant powder composition for making FaSSGF canine and FaSSIF canine

Two grams of the solid biorelevant powder composition for the preparation of FaSSGF *canine* and FaSSIF *canine* biorelevant medium is prepared by dissolving 0.727 g of sodium taurocholate (ST) and 0.711 g of sodium Taurodeoxycholate (STDC) in 10 ml of purified water at room temperature using a magnetic stirrer (alternatively the ST and the TSDC are added in separate steps). After the ST and the STDC are completely dissolved and a clear solution is obtained 10 ml of tert-butanol is added to the solution. In the next step 0.249 g of diacyl phospholipid and 0.198 g of monoacyl phospholipid is dissolved in the solution. After the lipid components are completely dissolved and a clear to slightly yellowish solution is obtained, 0.115 g of sodium oleate is added to the solution. The final clear to slightly yellowish solution is transferred into a suitable container for freeze-drying.

### Example 4:

### Making FaSSGF canine

0.149 g of a homogeneous powder composition from example 3 (comprising sodium taurocholate, sodium taurodeoxycholate, diacyl phospholipids, monoacyl phospholipids and sodium oleate) is dissolved in either a 1 liter of phosphate buffer comprising buffer and osmotic agents or 1 liter of a pH 1.5 non-buffered HCl solution comprising an osmotic component. The pH of the biorelevant medium is adjusted to either pH 6.5 or 1.5.

| | | |
|---|---|---|
| Sodium taurocholate | 0.1 mmol | 0.054 g/l |
| Sodium taurodeoxycholate | 0.1mmol | 0.053 g/l |
| Diacyl phospholipids | 0.025 mmol | 0.019 g/l |
| Monoacyl phospholipids | 0.025 mmol | 0.015 g/l |
| Fatty acids or monovalent salts | 0.025 mmol | 0.009 g/l |
| pH | **1.5 (±0.25) (dogs treated with Pentagastrin) and ***6.5 (±0.25) (dogs not treated without Pentagastrin) | |
| **Surface tension** | **35.0 mN/m** | |

### Example 5:

### Making FaSSIF canine

7.46 g of a homogeneous powder composition from example 3 (comprising sodium taurocholate, sodium taurodeoxycholate, diacyl phospholipids, monoacyl phospholipids and sodium oleate) is dissolved in 1 liter of phosphate buffer (example 11) comprising buffer and osmotic agents. The pH of the biorelevant medium is adjusted to pH 7.5.

| | |
|---|---|
| Sodium taurocholate | 5 mmol |
| Sodium taurodeoxycholate | 5 mmol |
| Diacyl phospholipids | 1.25mmol |
| Monoacyl phospholipids | 1.25 mmol |
| Fatty acids or monovalent salts | 1.25 mmol |
| pH: | 7.5 |
| **Surface tension** | **40.6 mN/m** |

### Example 6:

### FaSSIF Composition + Fatty Acids

| | | |
|---|---|---|
| Sodium taurocholate | 3 mmol | |
| Diacylphospholipids | 0.75 mmol | |
| Sodium oleate (phosphate buffer) | 0.75 mmolpH | 6.5 |
| **Surface tension** | **39.3 mN/m** | |

### Example 7:

### FaSSIF Composition with Monoacyl- and Diacylphospholipids

| | |
|---|---|
| Sodium taurocholate | 3 mmol |
| Diacylphospholipids | 0.075 mmol |
| Monoacylphospholipids | 0.675 mmol |
| pH | 6.5 (phosphate buffer) |
| **Surface tension** | **42.3 mN/m** |

### Comparative examples

### Example 8:

### FaSSIF V2 (Jantratid 2008)

| | |
|---|---|
| Sodium taurocholate | 3 mmol |
| Diacyl phospholipids | 0.2 mmol |
| pH | 6.5 (maleate buffer) |
| **Surface tension** | **54.3 mN/m** |

### Example 9:

### FaSSIF (WO 2007/054342)

| | |
|---|---|
| Sodium taurocholate | 3 mmol |
| Diacylphospholipids | 0.75 mmol |
| pH | 6.5 (phosphate buffer) |
| **Surface tension** | **53.3 mN/m** |

### Example 10:

### Blank Phosphate Buffer pH 6.5 for preparing FaSSIF

| | |
|---|---|
| Sodium dihydrogen phosphate | 28.65 mmol |
| Sodium hydroxide | 10.5 mmol |
| Sodium chloride | 105.85 mmol |
| pH | 6.5 |
| **Surface Tension** | **73.6 mN/m** |

### Example 11:

### Blank Phosphate Buffer pH 7.5 for preparing FaSSIF canine

| | |
|---|---|
| Sodium dihydrogen phosphate | 28.65 mmol |
| Sodium hydroxide | 21.66 mmol |
| Sodium chloride | 39.14 mmol |
| pH | 7.5 |
| **Surface Tension** | **72.9 mN/m** |

### Example 12

### Blank Maleate Buffer pH 6.5 for preparing FaSSIF human

| | |
|---|---|
| Maleic acid | 21.68 mmol |
| Sodium hydroxide | 40.23 mmol |
| Sodium chloride | 23.12 mmol |
| pH | 6.5 |
| **Surface Tension** | **73.4 mN/m** |

### Example 13

### HCl pH 1.5 for preparing FaSSGF canine

| | |
|---|---|
| Hydrochloric acid | q.s. pH 1.5 |
| Sodium chloride | 20.00 mmol |
| pH | 1.5 |
| **Surface Tension** | **72.8 mN/m** |

### Contribution by the Invention

The invention teaches optimized and standardized biorelevant media within the meaning of the definition, simulating fasted state conditions in the stomach and the upper small intestine of a given mammalian species including but not limited to human and canine models. Standardized biorelevant media is defined by (a) at least 40 mole % of at least one bile salt and the remaining mole % made up of at least two named surfactants and optional cosurfactants (b) mole ratio between the surfactants (c) mole concentration (mole %) of each surfactant and optional cosurfactant (d) total amounts (mole %) of all surfactants and optional cosurfactants in the media (e) pH, osmolarity and buffer capacity specific to the species and the region of the gastrointestinal tract.

Standardised biorelevant media described in this invention are further characterised by surface tension in the range between 25 mN/m and 50 mN/m. Within this range, the optimal surface tension for a given location in the intestinal tract of the mammalian species of interest can be provided by selecting combinations of at least one bile salt and at least two named surfactants selected from a group of surfactants present in the digestive tract of mammals and optional cosurfactants for determining the target surface tension value.

Non human FaSSGF and FaSSIF are described for the first time. Biorelevant media employed up to-date in early drug development studies in canine models for *in vitro -in vivo* correlation and prediction, for example FaSSGF, FaSSIF, FaSSIFv2, have actually been designed for human studies and are not optimised and standardised in terms of the proposed surface tension parameter in the range of 25mN/m to 50mN/m. The present invention provides canine biorelevant media that can be used for veterinary *in vitro* bridging assessments, thereby minimizing the number of *in vivo* studies in dogs. Further, provision of separate animal biorelevant media separates the preclinical data in animals from human clinical data, allows more objective comparisons/correlations and streamlines pharmaceutical development of drug products and post approval innovations/changes in formulation. Tests in separate biorelevant media which is species specific de-risks bioequivalence studies by affording the opportunity to fine tune the formulation to be tested.

The prior art has not considered targeting surface tension of the biorelevant media for *in vitro* testing. Surface tension is a desirable property because lowering the surface tension leads to an increase in contact ("wetting") between the biorelevant media and the surface of poorly soluble drug particles or drug products thereby facilitating dissolution. It is advantageous that the media be easily and reproducibly prepared in an efficient manner as this will lead to more reliable results and thereby better forecasting of *in vivo* drug performance. Moreover, there is an unmet need for standardised media characterised by a surface tension parameter within the overall range between 25mN/m and 50mN/m which can be implemented with assurance of reproducibility in laboratories around the world.

Exemplary embodiments below set out the invention.

Standardised aqueous biorelevant media for simulating fasted state stomach and fasted state upper small intestinal fluids of mammalian species,
composed of surfactants occurring in the gastro intestinal tract of mammals comprising
(a) at least 40 mole % to 95 mole % of one bile salt, and
(b) the rest mole % being a combination of at least two surfactants (excluding bile salt), namely
   - a diacyl phospholipid and a fatty acid including monovalent salts of the fatty acid, or
   - a monoacyl phospholipid and a fatty acid including monovalent salts of the fatty acid, or
   - a diacyl phospholipid and a monoacyl phospholipid and a fatty acid including monovalent salts of the fatty acid, or
   - a diacyl phospholipid and a monoacyl phospholipid,
further characterised by a surface tension between 25 mN/m and 50 mN/m.

The said biorelevant media wherein the surface tension is between 35 mN/m and 45mN/m, preferably between 28 mN/m and 45 mN/m and more preferably between 30 mN/m and 42 mN/m.

The said biorelevant media comprising at least 60 mole % and more preferably at least 70 mole % of at least one bile salt.

The said biorelevant media wherein the mole ratio of the two named surfactants in the mixture is 1:20 to 20:1.

The said biorelevant media further comprising between 0,001 mol % and 10 mole % co-surfactants naturally occurring in the gastro intestinal tract of mammals selected from the group consisting of cholesterol or their esters, monoglycerides, diglycerides, triglycerides, decomposition products of phospholipids other than fatty acids, and mixtures thereof.

The said biorelevant media wherein the mole ratio of the bile salts to the sum of the surfactants comprised in said combination of at least two surfactants and co-surfactants if present is 2 : 3 to 19 : 1, preferably 1 : 1 to 15 : 1, more preferably 2 : 1 to 6: 1 and most preferably 3 : 1 and 5: 1. In this context, if any of above-mentioned optional co-surfactants are present in the media, said co-surfactants are also accounted for in said sum.

The said biorelevant media wherein the mole ratio of said at least one monoacyl phospholipid and diacyl phospholipid to said fatty acids, including monovalent salts of fatty acids, in the mixture is 1:20 to 20:1.

The said biorelevant media wherein the mole ratio of diacyl phospholipids to monoacyl phospholipids in the mixture is 1:20 to 20:1.

The said biorelevant media wherein the mole ratio of diacyl phospholipids to fatty acids, including monovalent salts of fatty acids in the mixture is 1:20 to 20:1.

The said biorelevant media further comprising components selected from water, buffer, osmotic components, stabilizers, antioxidants, pH adjusters, antimicrobials, enzymes for example pepsin, pancreatic enzymes.

A homogeneous biorelevant composition for preparing fasted state biorelevant media characterised by a surface tension between 25 mN/m and 50 mN/m comprising the following surfactants:
(a) at least 40 mole % to 95 mole % of one bile salt, and
(b) the rest mole % being a combination of at least two surfactants, namely
   - a diacyl phospholipid and a fatty acid including monovalent salts of the fatty acid, or
   - a monoacyl phospholipid and a fatty acid including monovalent salts of the fatty acid, or
   - a diacyl phospholipid and a monoacyl phospholipid and a fatty acid including monovalent salts of the fatty acid, or
   - a diacyl phospholipid and a monoacyl phospholipid.

The said biorelevant composition for preparing human and mammalian FaSSIF and FaSSGF with a surface tension between 25mN/m and 50mN/ comprising at least 60 mole % and more preferably at least 70 mole % of the bile salt.

The said biorelevant composition in the form of a solid, for example powders wherein the mean particle size is between 10µm and 1000µm; bulk density is between 0.3 g/cm³ and 0.7 g/cm³ and moisture content is below 5 % by weight; or granules, pellets, or granules, pellets, tablets, or capsules wherein the mean particle diameter of the granules or pellets is 200 µm to 2000 µm.

The said biorelevant composition in the form of a liquid composition for example aqueous concentrates comprising between 10% and 60%; preferably 20 % to 60 % by weight of surfactants dispersed in aqueous medium comprising further preferably antioxidants and antimicrobials.

A method of preparing the said solid biorelevant composition comprising, dissolving the surfactants in a volatile solvent, water or mixtures thereof and eliminating the solvent, thereby providing a solid composition wherein the moisture content is below 5 % by weight.

A method of preparing a liquid biorelevant composition wherein between 10 % and 60 % by weight of the surfactants are homogeneously dissolved or dispersed in aqueous medium comprising further components selected from buffer, osmotic components, stabilizers, antioxidants, pH adjusters, and antimicrobials at a temperature between 15°C and 60°C without a drying step to remove the water.

A method for preparing FaSSIF *human* media comprising 2 to 20 mmol, preferably 2 to 6 mmol and FaSSIF *canine* media comprising 2 to 20 mmol preferably 10 to 15 mmol of the biorelevant compositions comprising a step for adding aqueous medium to the said solid or diluting the said liquid biorelevant compositions with the aqueous medium wherein the aqueous medium comprises buffers and osmotic regulators.

A method for preparing FaSSGF *human* media comprising between 0.01 mmol and 5 mmol, preferably 0.01 mmol and 1 mmol and FaSSGF *canine* media comprising between 0.1mm and 5 mmol, preferably 0.1 and 2 mmol of the biorelevant composition comprising a step for adding aqueous medium to the said solid or diluting the said liquid biorelevant compositions with the aqueous medium wherein the aqueous medium comprises buffers and osmotic regulators.

A method for preparing standardised biorelevant media according comprising individually weighing and dissolving the surfactants and optionally co-surfactants separately, together or sequentially in aqueous medium comprising components selected from water, buffer, osmotic components, stabilizers, antioxidants, pH adjusters, and antimicrobials and enzymes for example pepsin, pancreatic enzymes.

## Claims

1. Standardised aqueous biorelevant media for simulating fasted state stomach and fasted state upper small intestinal fluids of mammalian species,
composed of surfactants occurring in the gastro intestinal tract of mammals comprising
(a) at least 40 mole % to 95 mole % of one bile salt, and
(b) the rest mole % being a combination of at least two surfactants, namely
- a diacyl phospholipid and a fatty acid including monovalent salts of the fatty acid, or
- a monoacyl phospholipid and a fatty acid including monovalent salts of the fatty acid, or
- a diacyl phospholipid and a monoacyl phospholipid and a fatty acid including monovalent salts of the fatty acid, or
- a diacyl phospholipid and a monoacyl phospholipid,
further **characterised by** a surface tension between 25 mN/m and 50 mN/m.

2. The biorelevant media according to claim 1 wherein the surface tension is between 35 mN/m and 45 mN/M, preferably between 28 mN/m and 45 mN/m and more preferably between 30 mN/m and 42 mN/m.

3. The biorelevant media according to any of the preceding claims wherein the mole ratio of the two named surfactants in the mixture is 1:20 to 20:1.

4. The standardised biorelevant media according to any of the preceding claims further comprising between 0.001 mol % and 10 mole % co-surfactants naturally occurring in the gastro intestinal tract of mammals selected from the group consisting of cholesterol or their esters, monoglycerides, diglycerides, triglycerides, decomposition products of phospholipids other than fatty acids, and mixtures thereof.

5. The biorelevant media according to any of the preceding claims, wherein the mole ratio of the bile salts to the sum of the surfactants comprised in said combination of at least two surfactants and co-surfactants if present is 2 : 3 to 19 : 1, preferably 1 : 1 to 15 : 1, more preferably 2 : 1 to 6: 1 and most preferably 3 : 1 and 5: 1.

6. The biorelevant media according to any of the preceding claims wherein the mole ratio of said at least one monoacyl phospholipid and diacyl phospholipid to said fatty acids, including monovalent salts of fatty acids, in the mixture is 1:20 to 20:1.

7. The biorelevant media according to any of the preceding claims wherein the mole ratio of diacyl phospholipids to fatty acids, including monovalent salts of fatty acids in the mixture is 1:20 to 20:1.

8. The biorelevant media according to any of the preceding claims, further comprising components selected from water, buffer, osmotic components, stabilizers, antioxidants, pH adjusters, antimicrobials, enzymes for example pepsin, pancreatic enzymes.

9. A homogeneous biorelevant composition for preparing fasted state biorelevant media **characterised by** a surface tension between 25 mN/m and 50 mN/m comprising the following surfactants:
(a) at least 40 mole % to 95 mole % of one bile salt, and
(b) the rest mole % being a combination of at least two surfactants, namely
- a diacyl phospholipid and a fatty acid including monovalent salts of the fatty acid, or
- a monoacyl phospholipid and a fatty acid including monovalent salts of the fatty acid, or
- a diacyl phospholipid and a monoacyl phospholipid and a fatty acid including monovalent salts of the fatty acid, or
- a diacyl phospholipid and a monoacyl phospholipid.

10. The homogeneous composition according to claim 9 in the form of powders wherein the mean particle size is between 10µm and 1000µm; bulk density is between 0.3 g/cm³ and 0.7 g/cm³ and moisture content is below 5 % by weight; or granules, pellets, tablets, or capsules wherein the mean particle diameter of the granules or pellets is 200 µm to 2000 µm.

11. The homogeneous composition according to any one of claims 9 and 10 in the form of a liquid composition comprising between 10% and 60%; preferably 20 % to 60 % by weight of surfactants dispersed in aqueous medium comprising further preferably antioxidants and antimicrobials.

12. A method of preparing a homogeneous solid biorelevant composition according to any one of claims 9 to 11 comprising, dissolving the surfactants in a volatile solvent, water or mixtures thereof and eliminating the solvent, thereby providing a solid composition wherein the moisture content is below 5 % by weight.

13. A method of preparing a homogeneous liquid biorelevant composition according to any one of claims 9 and 11 wherein between 10 % and 60 % by weight of the surfactants are homogeneously dissolved or dispersed in aqueous medium comprising further components selected from buffer, osmotic components, stabilizers, antioxidants, pH adjusters, and antimicrobials at a temperature between 15°C and 60°C without a drying step to remove the water.

14. A method for preparing FaSSIF *human* media comprising 2 to 20 mmol, preferably 2 to 6 mmol and FaSSIF *canine* media comprising 2 to 20 mmol preferably 10 to 15 mmol of the biorelevant compositions of any one of claims 9 to 13 comprising a step for adding aqueous medium to the homogeneous solid or diluting the liquid compositions with the aqueous medium wherein the aqueous medium comprises buffers and osmotic regulators.

15. A method for preparing FaSSGF *human* media comprising between 0.01 mmol and 5 mmol, preferably 0.01 mmol and 1 mmol and FaSSGF *canine* media comprising between 0.1mmol and 5 mmol, preferably 0.1 and 2 mmol of the biorelevant composition of any one of claims 9 to 13 comprising a step for adding aqueous medium to the homogeneous solid or diluting the liquid compositions with the aqueous medium wherein the aqueous medium comprises buffers and osmotic regulators.

16. A method for preparing biorelevant media according to any one of claims 1 to 8 comprising individually weighing and dissolving the surfactants and optionally co-surfactants separately, together or sequentially in aqueous medium comprising components selected from water, buffer, osmotic components, stabilizers, antioxidants, pH adjusters, antimicrobials and enzymes for example pepsin, pancreatic enzymes.
